Europäisches Patentamt

European Patent Office

Office européen des brevets

(19)

(11) Publication number: **0 238 971**
**A2**

(12)
# EUROPEAN PATENT APPLICATION

(21) Application number: 87103824.6

(22) Date of filing: 17.03.87

(51) Int. Cl.⁴: **C12P 21/00 , G01N 33/577**

(30) Priority: 17.03.86 US 840229

(43) Date of publication of application:
**30.09.87 Bulletin 87/40**

(84) Designated Contracting States:
**AT BE CH DE FR GB IT LI NL**

(71) Applicant: **F. HOFFMANN-LA ROCHE & CO.
Aktiengesellschaft**

**CH-4002 Basel(CH)**

(72) Inventor: **Chizzonite, Richard A.
10 Barnsdale Road
Clifton, N.J. 07013(US)**

(74) Representative: **Riederer Freiherr von Paar zu
Schönau, Anton et al
Van der Werth, Lederer & Riederer Freyung
615 Postfach 2664
D-8300 Landshut(DE)**

(54) **Antibodies directed to a lymphokine.**

(57) The invention is directed to monoclonal antibodies against IL-2 which have utility as affinity reagents for the purification of human and mouse IL-2 and as reagents for a sensitive two-site immunoassay. The invention is further directed to the use of the novel monoclonal antibodies of the invention as an affinity matrix to purify IL-2 and to the use of the monoclonal antibodies of the invention in a Two-site Sandwich Assay to quantify IL-2 in a biological sample, e.g. serum or plasma.

EP 0 238 971 A2

## ANTIBODIES DIRECTED TO A LYMPHOKINE

Within the last decade, it has become clear that immunocompetent cells produce both antigen-specific and nonspecific molecules which enable them to communicate with other immunocompetent cells in the generation and control of the immune response (Waksman, B.H. [1980] In Pick, E. [ed.]: Lymphokine Reports, Vol. I, New York Academic Press, 1; Germain, R.N. [1980] in Pick, E. [ed.]: Lymphokine Reports, Vol. I, New York, Academic Press, 7). When such molecules are secreted by B or T-lymphocytes, they are termed lymphokines; when produced by macrophages or monocytes, they are called monokines. These protein and glycoprotein molecules, which are secreted by cells and which act on other cells in close proximity to them, have been classified as cytokines. This type of local cell regulation by protein and peptide molecules has been termed autocrine to distinguish this type of cellular regulation from the endocrine system where mediators are carried by the blood and act on target tissues at distinct sites.

Interleukin-2 (IL-2) is one of a number of lymphokines which play a critical role in the regulation of immune responses. It was first identified as a discrete entity by Morgan, Ruscetti, and Gallo in 1976 (Morgan, D.A., Ruscetti, F.W., and Gallo, R [1976] Science 193, 1007). Previously known as T-cell growth facator (TCGF), IL-2 is secreted by T-lymphocytes after exposure to mitogens or antigens. It is this second signal which further stimulates proliferation of T-lymphocytes. This glycoprotein molecule is intimately involved in the induction and sustenance of virtually all immune responses in which T-cells pay a role (Farrar, J.J., Benjamin, W.R., Hilfiker, m.L., Howard, M., Farrar, W.L., and Fuller-Farrar, J. [1983] Immunol. Rev. 63, 129). IL-2 is believed to provide a universal signal for the proliferation of mature T-cells through its binding to specific cell-surface receptors (Smith, K.A. (198_) Immunol. Rev., 51:337-357; Robb et al. [1981] J. Exp. Med. 154:1455-1474). IL-2 has been shown to induce antigen-specific cytotoxic T-lymphocytes, natural killer cells, or lymphokine-activated killer cells, all of which are implicated as effector cells in surveillance against malignancy, to reconstitute functional T-cell responses and to assist in the immune T-cell-and chemotherapy-induced elimination of establish murine syngeneicleukemias (Altman et al. [1984] Proc Natl Acad Sci USA, 81:2176-2180).

Abnormalities in IL-2 production and/or response are associated with several diseases. Such defects have been identified in humans with systemic lupus erythematosus, in aged individuals, in children with primary immunodeficiencies, in bone marrow transplant recipients undergoing a graft versus host reaction and in parasite infected mice. (Altman et al. [1984] Proc Natl Acad Sci USA, 81:2176-2180). IL-2 may be useful as an immunological response modifier in cancer patients (Key et al., Immunology Letters [1983] 6:175-178).

Systemic administration of autologous lymphokine -activated Killer (LAK) cells and recombinantly derived IL-2 to 25 advanced metastatic cancer patients resulted in regression of the cancer in 11 of the 25 patients (Rosenberg et al., The New England Journal of Medicine [1985], 313:1485-1492).

In 1983, the entire primary sequence of Interleukin 2 was described by Taniguchi et al. (Taniguchi et al., Nature (London), [1983] 302:305-310). IL-2 contains 133 amino acids and has the following sequence:

```
 1                                        10
Ala-Pro-Thr-Ser-Ser-Ser-Thr-Lys-Lys-Thr-Gln-Leu-Gln-Leu-Glu-
                    20                                   30
His-Leu-Leu-Leu-Asp-Leu-Gln-Met-Ile-Leu-Asn-Gly-Ile-Asn-Asn-
                                    40
Tyr-Lys-Asn-Pro-Lys-Leu-Thr-Arg-Met-Leu-Thr-Phe-Lys-Phe-Tyr-
                    50                                   60
Met-Pro-Lys-Lys-Ala-Thr-Glu-Leu-Lys-His-Leu-Gln-Cys-Leu-Glu-
                                         70
Glu-Glu-Leu-Lys-Pro-Leu-Glu-Glu-Val-Leu-Asn-Leu-Ala-Gln-Ser-
                    80                                   90
Lys-Asn-Phe-His-Leu-Arg-Pro-Arg-Asp-Leu-Ile-Ser-Asn-Ile-Asn-
                                   100
Val-Ile-Val-Leu-Glu-Leu-Lys-Gly-Ser-Glu-Thr-Thr-Phe-Met-Cys-
                   110                                  120
Glu-Tyr-Ala-Asp-Glu-Thr-Ala-Thr-Ile-Val-Glu-Phe-Leu-Asn-Arg-
                                   130        133
Trp-Ile-Thr-Phe-Cys-Gln-Ser-Ile-Ile-Ser-Thr-Leu-Thr.
```

The amino acid and the corresponding three-letter designation and single-letter designation are as follows:

| Amino Acid | 3-Letter | 1-Letter |
|---|---|---|
| Alanine | Ala | A |
| Arginine | Arg | R |
| Asparagine | Asn | N |
| Aspartic Acid | Asp | D |
| Cysteine | Cys | C |
| Glutamic Acid | Glu | E |
| Glutamine | Gln | Q |
| Glycine | Gly | G |
| Histidine | His | H |
| Homoserine | Hse | – |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

Although it may theoretically be possible to synthetically produce IL-2, this method is not yet feasible for the production of quantities of homogeneous material. IL-2 is presently being produced recombinantly in fermentation culture. Monoclonal antibodies are useful analytical reagents for the large scale production and purification of IL-2.

In 1981, Gillis et al. (Gillis et al., the J of Immunol, [1981] 126:1978-1984) reported the production of a B cell hybridoma whose antibody product inhibited IL-2 activity. BALB/c female mice were immunized with IL-2 isolated from rat splenocytes. The spleens of the immunized mice were removed and single cell suspensions were produced. These spleen cells were fused with the BALB/c myeloma SP2/O. A monoclonal B cell hybridoma was isolated whose IgG product appeared to be directed against a determinant present on molecules of human, mice and rat IL-2 (Altman et al. [1984] Proc Natl Acad Sci USA, 81:2176-2180).

Stadler et al (Stadler et al., The J. of Immunol, [1982] 128:1620-1624) immunized BALB/c mice with partially purified human IL-2. The spleens of the immunized rats were harvested and the spleen cells were hybridized with plasmacytoma cells. Supernatants of the hybridoma cultures were screened for their ability to inhibit IL-2 proliferation of the CT6 cell line. Eight different lines were found to produce antibodies which inhibited the proliferation of the Il-2 dependent cell line in response to either crude or purified human IL-2 as well as rat and mouse IL-2. These anti-IL-2 antibodies did not inhibit the proliferation of human T cell lines capable of producing IL-2.

Smith et al (Smith et al., The J. of Immunol [1983] 131:1808-1814) disclose the preparation of three monoclonal antibodies to IL-2 which are identified as DMS-1, DMS-2 and DMS-3. DMS-1 and 2 reacted specifically with IL-2 as demonstrated by antibody concentration-dependent neutralization of IL-2 activity. DMS-3, although less effective in neutralizing IL-2, bound to IL-2 more avidly and functioned as an immunoabsorbent.

Altman et al (Altman et al., Proc Natl Acad Sci USA; 81:2176-2180, 1084) describe the preparation of polyclonal antibodies to human IL-2, using as immunogens, eight synthetic peptides devised from the predicted amino acid sequence of IL-2. Each peptide consisted of 13-15 amino acids and were derived from the IL-2 sequence data published by Taniguchi et al. (Taniguchi et al., Nature (London), [1983] 302:305-310). Antibodies to four of the eight peptides were found. An affinity-purified antibody to one of the IL-2 peptides specifically stained the cytoplasma of phytohemagglutinin-stimulated human peripheral blood lymphocytes. None of these antibodies demonstrated neutralization of IL-2 activity.

Robb et al. (Proc Natl. Acad. Sci. USA [1983] 80:5990-5994) reported the preparation of a monoclonal antibody designated IH II-IA5 which specifically reacted with the carbohydrate residue at amino acid 3 of IL-2. This antibody was used for affinity purification of IL-2 but did not inhibit the biological activity of IL-2.

Monoclonal antibodies to IL-2 provide powerful analytical and diagnostic tools for the study of IL-2 biological activity as well as the immunopurification of IL-2, the development of IL-2 immunoassays, the identification of the active site of the IL-2 molecule, and an assay of in vivo studies exploring the physiologic role of IL-2 in the immune response. Although monoclonal antibodies to IL-2 are known, few are available which may be routinely utilized as affinity reagents for the purification of IL-2 and as reagents in a sensitive two-site immunoassay for IL-2.

The present invention is directed to monoclonal antibodies against IL-2 which have utility as affinity reagents for the purification of human and mouse IL-2 and as reagents for a sensitive two-site immunoassay. The invention is further directed to the use of the novel monoclonal antibodies of the invention as an affinity matrix to purify IL-2 and to the use of the monoclonal antibodies of the invention in a two-site immuunoassay to quantifying IL-2 in a biological sample, e.g. serum or plasma.

The monoclonal antibodies of this invention in general have greater than $3,5 \times 10^7$ M$^{-1}$ affinity for native or recombinant human IL-2.

The monoclonal antibodies against IL-2 according to the present invention are prepared by forming hybridomas between antibody-producing cells of animals immunized with recombinant human interleukin 2 (rIL-2) and myeloma cells, cloning said hybridomas to select anti-human interleukin 2 antibody-producing clones and cultivating the thus selected clones producing anti-human interleukin 2 monoclonal antibodies.

The invention is specifically directed to monoclonal antibodies specific to native IL-2, recombinant IL-2, and IL-2 synthetic peptides. The invention is further directed to three groups of monoclonal antibodies having the following characteristics:

Group 1. A monoclonal antibody which inhibits IL-2 function to greater than 50% in both the IL-2 Bioassay and the Receptor Binding Assay and which binds (i.e. recognizes the epitopes) between amino acids 1-12, 9-19, 41-55 or 21-123 on the native or recombinant human IL-2 protein.

Group 2. A monoclonal antibody which has high affinity for native or recombinant human IL-2 as measured by IL-2 RIA assay, which does not inhibit IL-2 function when measured by the IL-2 Bioassay and Receptor Binding Assay and which binds between amino acids 40-70, 42-56, 21-123, 78-87, 52-70, 66-87, 107-121 or 7l-87 on the native or recombinant human IL-2 protein.

Group 3. A monoclonal antibody which inhibits native or recombinant mouse and human IL-2 in the IL-2 Bioassay and the Receptor Binding Assay and which binds between amino acids 41-55 on the IL-2 protein.

The monoclonal antibodies (mABs) of Group 1 inhibit IL-2 function to greater than 50% in the IL-2 Neutralization Assay and in the Receptor Binding Assay. The mABs Group I recognize or bind an epitope located at amino acid 1-12, 9-19, 41-55 or 21-123 on the native or recombinant IL-2 protein. These mAB's can be used to identify two separate amino acid sequences which are necessary for the bioactivity of IL-2; i.e., sequences 1-19 and 41-55.

The monoclonal antibodies of Group 2 do not inhibit IL-2 function. Group 2 monoclonal antibodies of the invention have a high affinity ($10^7$-$10^9$ M$^{-1}$) for IL-2 when measured by the Radio Immunoassay and, thus, are useful as reagents for affinity chromatography to purify or isolate human IL-2 and also for antibodies in the Two Site Immunoassay. Group 2 mABs identify amino acid sequences which are not necessary for the bioactivity of IL-2.

The monoclonals of Group 3, inhibit both mouse IL-2 and human IL-2 function in the IL-2 Neutralization Assay and Receptor Binding Assay. The mABs of Group 3 are particularly useful as model antagonists of IL-2 action in both in vitro and in vivo assays. Further, mAB's in this group are particularly suited for incorporation into an affinity matrix for the purification of human or mouse IL-2 and especially for the large scale production of mouse IL-2 which is needed for pre-clinical studies.

The term Enzyme Immunoassay ("EIA") refers to the following assay. Hybridoma supernatents are reacted with either r-IL-2 or IL-2 synthetic peptides immobilized on a solid substrate, e.g., a microtiter plate. Following sequential incubations with goat anti-mouse IgG coupled to peroxidase and o-phenylenediamine, the amount of antibody bound to r-IL-2 or IL-2 peptide is determined by reading the optical density (OD 488) of each well. A positive result is scored if the specific binding is greater than $10^x$ background value.

The term "Neutralization Assay" refers to a conventional T-cell growth assay as described by Gillis et al. (Gillis et al., [1978] J. Immunol 120:2027). Minor modifications to the standard procedure are within the skill of the art and may be appropriate depending upon individual requirements. Briefly, an IL-2 dependent cloned murine cytotoxic T-cell line is incubated in the presence of IL-2 and the monoclonal antibody to be tested. If the monoclonal antibody binds to a "neutralizing" epitope on the IL-2 protein, IL-2 will not be available for uptake by the T-cell line; that is, the monoclonal antibody "neutralizes" the IL-2 activity. If the mAB does not bind to a neutralizing epitope on the IL-2 protein, IL-2 will be available for uptake by the T-cell line.

The term "Binding Assay" refers generally to a procedure developed by Robb et al. (Robb et al. [1981] J. Exp. Med. 154:1455-1474). As used herein, the term specifically refers to an assay which measures the ability of the monoclonal antibody to inhibit the binding of radiolabelled IL-2 ($^{125}$I-IL-2) to receptor sites on target cells.

The term "epitope" (sometimes referred to as an "antigenic determinant") is that area of the IL-2 molecule to which the monoclonal antibody of the invention binds. This "area of the IL-2 molecule" is the amino acid sequence recognized by the mAB. According to convention, the amino acid in the IL-2 protein are numbered starting from the N-terminal amino acid; thus, the amino acid segment 1-12 is the first twelve amino acids of the IL-2 protein.

The term "Radio Immunoassay" (RIA) refers to an assay which measures the ability of the monoclonal antibodies of the invention to bind to $^{125}$I labelled IL-2. The monoclonal antibodies of the invention are incubated with goat anti-mouse IgG coupled to a solid (agarose beads) substrate. The beads are pelleted by centrifugation and the pellet is incubated with $^{125}$I labelled IL-2. Thereafter, the radioactivity of the pellet is counted using a gamma counter. The greater the affinity of the mAB for the $^{125}$I-labelled IL-2 the higher will be the gamma count.

Peptides were derived from the published sequence of the amino acid sequence of human IL-2 (Barany, G. and Merriefield, R. B. [1980] in the peptides:Analysis, Synthesis, Biology, Eds. Gross. E. and Meienhofer, J. Academic press, New York Vol. 2 pp 1-255). Synthetic peptides were prepared by the Merrifield solid-phase method (Tam. J. P., Heath, W. F. and Merriefield, R. B. [1983] J. Am. Chem. Soc. 105, 6442-6455). The composition of all peptides was confirmed by amino acid analysis. The Amino acid sequence of the synthetic peptides used to probe the hybridoma supernatants are shown in Table 1.

Recombinant IL-2 analogs were prepared as follows.

Defined amino acid changes were made by mutagenesis of a double-stranded DNA plasmid capable of expressing human IL-2 protein in E. coli. The procedure for site-specific mutagenesis utilizes synthetic oligonucleotides which code for amino acid changes and was performed as described by Morinaga et al. (Biotechnology 2:636-639 1984). For each mutation, 100 ng of gapped and linear plasmid DNA were mixed with 1 pmole of phosphorylated synthetic oligonucleotide. The DNA's were denatured by heating, then allowed to anneal at room temperature. The single-stranded region of the heteroduplex molecules which formed was then filled in, incorporating the synthetic oligonucleotide. A sample of the DNA mixture was then transferred into E. coli strain MC1061. Bacterial colonies containing the mutated plasmid were identified by specific hybridization to the $^{32}$P-labelled synthetic oligonucleotide under conditions in which the wild-type IL-2 DNA did not hybridize. The mutated plasmid was induced to express the recombinant IL-2 protein analogue by growth of the host cells at 42°C under standard conditions.

The analogs in which single amino acid charges were made are shown in Table 1a. Additionally, several deletion mutants were prepared in which amino acids 1-10, 1-20 and 124-133 were deleted from the native protein.

Table 1: Amino acid sequence of the synthetic peptides

```
                                          10                                              20
H-Ala-Pro-Thr-Ser-Ser-Ser-Thr-Lys-Lys-Thr-Gln-Leu-Gln-Leu-Glu-His-Leu-Leu-Leu-Asp
                                          30                                              40
Leu-Gln-Met-Ile-Leu-Asn-Gly-Ile-Asn-Asn-Tyr-Lys-Asn-Pro-Lys-Leu-Thr-Arg-Met-Leu
                                          50                                              60
Thr-Phe-Lys-Phe-Tyr-Met-Pro-Lys-Lys-Ala-Thr-Glu-Leu-Lys-His-Leu-Gln-Cys-Leu-Glu
                                          70                                              80
Glu-Glu-Leu-Lys-Pro-Leu-Glu-Glu-Val-Leu-Asn-Leu-Ala-Gln-Ser-Lys-Asn-Phe-His-Leu
                                          90                                              100
Arg-Pro-Arg-Asp-Leu-Ile-Ser-Asn-Ile-Asn-Val-Ile-Val-Leu-Glu-Leu-Lys-Gly-Ser-Glu
                                          110                                             120
Thr-Thr-Phe-Met-Cys-Glu-Tyr-Ala-Asp-Glu-Thr-Ala-Thr-Ile-Val-Glu-Phe-Leu-Asn-Arg
                                          130       133
Trp-Ile-Thr-Phe-Cys-Gln-Ser-Ile-Ile-Ser-Thr-Leu-Thr-OH.
```

Contiguous Peptides

| 1-12 | 13-26 | 27-41 | 42-56 | 57-70 | 71-87 | 88-105 | 105-121 | 122-133 |

9-26

23-41

36-56

52-70

66-87

84-105

98-121

Overlap Peptides

116-133

Table 1a: Recombinant IL-2 Analogs

| Position of Amino Acid (AA) In Native IL-2 | 3 | 8 | 9 | 12 | 13 | 14 | 15 | 16 | 17 | 18 | 19 | 20 | 41 | 42 | 43 | 45 | 46 | 48 | 49 | 50 | 54 | 55 | 58 |
|---|---|---|---|---|---|---|---|---|---|---|---|---|---|---|---|---|---|---|---|---|---|---|---|
| AA in Native Protein | Thr | Lys | Lys | Leu | Gly | Leu | Glu | His | Leu | Leu | Leu | Asp | Thr | Phe | Lys | Tyr | Met | Lys | Lys | Ala | Lys | His | Cys |
| AA in Analog | -Tyr | Glu | Glu | Thr | Lys | Thr | Lys | Asp | Asn | Thr | Thr | Asn | Val | Tyr | Glu | Phe | Leu | Glu | Glu | Trp | Ala | Asp | Ser |

In order to achieve high titers of anti-IL-2 antibodies in the serum, it is important to use between about 25 to about 50µg of immunogen, preferably about 50µg of immunogen at day 0 and 7 followed by booster immunization of from about 50µg to about 75µg of immunogen at day 30 preferably about 75µg of immunogen to obtain EIA and RIA titers of from about 1:0.5-10 $^x$ 10$^5$ and about 1:1-2 $^x$ 10$^3$ dilution respectively.

The following examples provide detailed description of the preparation and use of the monoclonal antibodies of the invention.

## Example 1

### Immunizations

Eight female Balb/c mice (Charles River Laboratories, Wilmington, Mass.), 8-10 weeks of age, were immunized with 25 to 50µg of recombinant human interleukin 2 (rIL-2) in complete Freund's adjuvant on days 0 and 7. The immunizations were at three sites: one intraperitoneal injection (i.p.) and two subcutaneous injections into the left and right inguinal region. On day 30 the mice were bled via the retro-orbital plexus and their sera were tested for anti-IL-2 antibodies in an enzyme immunoassay (EIA) and a radioimmunoassay (RIA). The mice were given an intraperitoneal booster immunization of 50-75µg of rIL-2 on day 60 and were bled again on day 90. The sera were tested for anti-IL-2 antibodies and subsequently for ability to inhibit the bioactivity of IL-2. The inhibition of IL-2 bioactivity was evaluated by two methods: 1) neutralization of IL-2 dependent growth of mouse CTLL cells or human peripheral blood lymphocyte blasts, (Neutralization assay) and 2) inhibition of binding of $^{125}$I-labelled IL-2 to target cells (Receptor Binding Assay). Both assays measure high affinity antibodies which bind epitopes on IL-2 that are necessary for IL-2 to bind the cellular receptor. The sera from day 90 demonstrated EIA and RIA titers of approximately 1:0.5 -1.0 $^x$ 10$^5$ and 1:1-2 $^x$ 10$^3$ dilution, respectively, and also inhibited the bioactivity of IL-2. Forty five to 100 days after the second bleed, the mice were given an intravenous (i.v.) and an i.p. booster immunization on each of three successive days with 50µg of rIL-2. On the fifth day, the mice were killed and the spleens removed for preparation of splenocytes.

## Example 2

### Preparation of Hybridoma Cell Fusions

Two days before fusion, splenocyte feeder cells were prepared from native mice in complete medium [IMDM + 10% fetal bovine serum, glutamine (2.0 mM), and 2-mercaptoethanol (100 µM) containing 100 uM hypoxanthine, 0.4 µM aminopterine, and 16 µM thymidine (HAT)]. Following a modification of the procedure of De St.Groth and Scheidegger, (1980) J. Immunol. Methods. 35:1, spleen cells (10$^5$) were fused with 10$^5$ PAI-0 mouse myeloma cells (J.W. Stocker et al., Research Disclosure, 217, May 1982, pp. 155-157) growing in logarithmic phase. The cells were mixed, pelleted by centrifugation and resuspended under constant gentle agitation in 1.0 ml of 35% (v/v)) polyethylene glycol in IMDM at 37°C over 1 min. After incubation for 3 min. at 37°C, the cells were pelleted again by centrifugation and gently resuspended in 10 ml of IMDM + HAT. The cells were then diluted to 1 $^x$ 10$^5$ cells per ml in complete medium + HAT and dispersed to 24 well microtiter plates (1 ml/well) containing 5 $^x$ 10$^5$ feeder cells in 1 ml of complete medium. Hybridoma supernatents were assayed for anti-IL-2-antibodies by the IL-2 EIA and RIA as shown in Examples 3 and 4. Hybridomas were cloned by limiting dilution.

## Example 3

### IL-2 EIA

Hybridoma culture supernatents (65 µl) were added to wells of microtiter plates (Falcon Probind or Costar EIA) which had been coated with either rIL-2 or with the IL-2 peptides of Table 1, (100 ng/well at 37°C overnight) and which contained 35µl of 2% BSA in 25mM sodium phosphate pH 6.5 + 1 M NaCl + 0.15% Tween 20. After incubation at room temperature for 2 hrs, the wells were washed with PBS (25 mM sodium phosphate pH 7.4, 0.15 m NaCl) + 0.05% Tween 20. One hundred $\mu l$ of goat anti-mouse IgG + IgM coupled to horseradish peroxidase (Boehringer-Mannheim Biochemicals) (1:1000 dilution in antibody buffer: 25 mM sodium phosphate pH 6.5, 0.5 M NaCl + 0.05% Tween 20)) was added to each well and incubated for 90 min. at room temperature. The wells were washed with PBS + 0.05% Tween 20, and incubated with o-phenylenediamine (0.4 mg/ml in 0.1 M citrate buffer pH 4.5 + 0.012% hydrogen peroxide) for 30 min. at room temperature. The reaction was stopped by the addition of $50\mu l$ of 2.5 M $H_2SO_4$ containing 50 mM sodium metabisulfite. The $OD_{488}$ of the substrate color was read on a Titertek Multiscan MC.

## Example 4

### IL-2 RIA

Hybridoma supernatents (0.1 - 0.5 ml) were incubated with 100 $\mu l$ of a 50% suspension of goat anti-mouse IgG coupled to agarose beads (Sigma Chemical Co.) in 1% BSA in RIPA buffer (50mM sodium phosphate pH 7.5, 1% Triton X-l00, 1% deoxycholate, 0.1% sodium dodecylsulfate, 0.15M NaCl, 5mM EDTA) for 2 hrs on a rotating mixer at room temperature. The beads were pelleted by centrifugation, washed $1^x$ in RIPA buffer, and 250 $\mu l$ of $^{125}I$ labelled IL-2 (10-20 fmoles, $1 - 2 \times 10^4$ cpm) in 1% BSA + RIPA buffer was added. After overnight incubation at 4°C on a rotating mixer, the beads were washed with RIPA buffer and counted in an LKB gamma counter.

## Example 5

### IL-2 Neutralization Assay

IL-2 bioactivity was determined by a microassay with the use of an IL-2 dependent cloned murine cytotoxic T cell line (CTLL) as described by Gillis et al. (Altman et al. [1984] Proc Natl Acad Sci USA, 81:2176-2180; Robb et al., Proc Natl. Acad. Sci. USA [1983] 80:5990-5994. Inhibition of IL-2 bioactivity was assayed by a modification fo the IL-2 activity assay. Briefly, $25\mu l$ of concentrated hybridoma supernatent ($20^x$) was incubated with 25 $\mu l$ of media containing IL-2 (1 Unit/ml) for 1 hr at 37°C. This mixture was added to 50 $\mu l$ of media and CTLL cells in a microtiter plate and incubated overnight at 37°C. The next day the cultures were pulsed with $^3H$-thymidine (0.5 $\mu Ci/50\mu l$/well) and incubated for 5 hrs at 37°C. The incorporation of $^3H$-thymidine was determined by liquid scintillation counting, and the results are expressed as the percent of inhibition of $^3H$-thymidine incorporation in the presence of antibody compared to medium control.

## Example 6

### IL-2 Receptor Binding Assay

Inhibition of Binding of $^{125}I$-IL-2 to target cells by hybridoma antibodies was determined as described (Robb, R.J. et al. 1981 loc. cit.) with some modifications. Hybridoma supernatents ($20^x$ concentrate) were preincubated with $^{125}I$-IL-2 for 1 hr at 37°C. One hundred twenty $\mu l$ aliquots of murine CT6 of human peripheral blood lymphocyte blast cells ($6 \times 10^4$ cells) were added to give a final volume of $150\mu l$. After incubation for 20 min at 37°C, the cells were centrifuged for 90 sec at 4°C through a silicone oil mixture. Tube tips containing the cell pellets were excised and radioactivity determined in a gamma counter. The results are expressed as percent inhibition of $^{125}I$-IL-2 bound in the presence of antibody compared to control values. Table 2 summarizes the results of the assays described in Example 3-6. The amino acid in parenthesis, e.g. "(Lys 8)" under the column labelled "epitope" is essential for binding to the monoclonal antibody.

Table 2

Comparison of the Functional Domains of IL-2 and Epitopes of Monoclonal Anti-IL2 Antibodies

| MONOCLONAL ANTIBODY | | EPITOPE | EIA BINDING | | INHIBITION OF IL-2 FUNCTIONAL ASSAYS | | | | RIA[3] | AFFINITY |
|---|---|---|---|---|---|---|---|---|---|---|
| Group | mAB# | AA Sequence | HU | MS | Neutralization[1] | | | Binding[2] | $^{125}$I-II-IL-2 (%) | (M$^{-1}$) |
| | | | | | H | M | R | H | | |
| No.1 – Inhibit human IL-2 function | 5B1 | 1–12 (Lys 8) | + | – | 100 | 0 | 0 | 100 | 100 | 1.2X10$^9$ |
| | 7B1 | 9–19 (His 16) | + | – | 100 | 0 | 0 | 100 | 92 | – |
| | 5A5 | 21–123 | + | – | 100 | 0 | 0 | 91 | 78 | 3.5x10$^7$ |
| | 13D2 | 41–55 | + | – | 50 | 0 | 0 | 60 | 100 | 5.2x10$^7$ |
| No.2 – Do not inhibit IL-2 function | 1A4 | 40–70 | + | – | 0 | ND | ND | 0 | 92 | – |
| | 16A4 | 42–56 | + | – | 0 | ND | ND | 0 | 80 | – |
| | 31A4 | 40–70 | + | + | 0* | 0 | ND | 0* | 100 | 2.8x10$^7$ |
| | 17A1 | 21–123 | + | – | 0* | 0 | ND | 0* | 100 | 1.1x10$^9$ |
| | 9A2 | 78–87 | + | – | 0* | 0 | ND | 0* | 100 | – |
| | 8D1 | 52–70 | + | – | 0 | ND | ND | 0 | 86 | – |
| | 12B5 | 66–87 | + | – | 0 | ND | ND | 0 | 63 | – |
| | 3D5 | 71–87 | + | – | 0 | ND | ND | 0 | 100 | 2.9x10$^8$ |
| | 4A4 | 107–121 | + | + | ND | ND | ND | ND | ND | – |
| No.3 – Inhibits mouse and human IL-2 function | 13A6 | 41–55 | + | + | 40 | 75 | 0 | 12 | 94 | 3.6x10$^7$ |

1. The results are expressed as the percent inhibition of $^3$H-thymidine incorporation in the presence of antibody when compared to control values.

2. The results are expressed as the percent inhibition of $^{125}$I-IL-2 bound in the presence of antibody when compared to control values.

3. The results are expressed as the percentage of total $^{125}$I-IL-2 bound by the antibody when compared to control value. Total $^{125}$I-IL-2 input was 20 fmoles.

H, M, R, - Neutralization assays performed with either human (H$_2$), mouse (M), or rat (R) IL-2.

*  Assays performed at a 1000 fold molar excess of antibody to IL-2.
ND Not determined.
AA Amino Acid

The epitopes of the hybridoma antibodies were identified by EIA reactivity on the IL-2 synthetic peptides described above and by Western blot and dot blot analysis on recombinant IL-2 analogues of Table 1. The Western Blot and Dot Blot assays are described in Example 7.

Example 7

Epitope Mapping Analysis

For Western blots, recombinant human IL-2 analogous, an E. coli protein extract is separated by SDS-PAGE, transferred to nitrocellulose and probed with each hybridoma antibody. For Dot Blots, the E. coli extracts are applied directly to nitrocellulose, dried and probed with each antibody. The antibody bound to the IL-2 analog is visualized with a peroxidase conjugated anti-antibody and the peroxidase substrate, 4-Cl-1-napthol. The results are summarized in Table 3 for the 40-70 amino acid region of IL-2. The data shown in Table 3 is a composite of the reactivity of each mAB with different IL-2 analogs as determined by Western and Dot blot analysis.

TABLE 3

## Micro-Epitope Analysis Within the 40-70 Amino Acid Region of IL-2

| | Monoclonal Antibody Effect on IL-2 Function | | | | |
| | Inhibitory | | Non-Inhibitory | | |
| Peptides Bound | 13D2 | 13A6 | 1A4 | 16A4 | 31A4 |
|---|---|---|---|---|---|
| 30-70 | + | + | + | + | + |
| 40-70 | + | + | + | + | + |
| 42-56 | + | + | − | + | − |
| 52-70 | − | − | − | − | − |

IL-2 Analogues Bound

| | 13D2 | 13A6 | 1A4 | 16A4 | 31A4 |
|---|---|---|---|---|---|
| $Thr^{41} \to Val$ | + | − | + | + | − |
| $Phe^{42} \to Tyr$ | + | + | + | + | + |
| $Lys^{43} \to Glu$ | − | − | − | − | − |
| $Tyr^{45} \to Phe$ | + | − | + | − | − |
| $Met^{46} Leu$ | + | + | + | + | + |
| $Lys^{48} \to Glu$ | − | − | − | − | − |
| $Lys^{49} \to Glu$ | + | + | + | + | + |
| $Ala^{50} \to Trp$ | + | + | + | + | + |
| $His^{55} \to Asp$ | − | + | − | + | − |
| $Cys^{58} \to Ser$ | + | + | + | + | + |
| Mouse IL-2 | − | + | − | − | ± |

The monoclonal antibodies of the invention may be used as reagents in conventional affinity chromatography procedures. The monoclonal antibodies of Group 3 are particularly useful as affinity reagents as they may be used to isolate and purify both mouse and human IL-2. The monoclonal antibodies described herein are also useful as reagents in a "Two-Site Sandwich Assay" to measure dilute quantities of IL-2 in biological fluids, e.g. serum or plasma.

The monoclonal antibodies of the invention are useful in a method for determining the amount of IL-2 protein in a sample of biological fluid, which method comprises the steps of:

(1) binding to a solid carrier a first monoclonal antibody having the ability to bind to an epitope in the IL-2 protein;

(2) contacting a sample of biological fluid with said first monoclonal antibody to form an insoluble complex of said peptide and said IL-2 protein in said sample;

(3) contacting the complex of Step 2 with a measured amount of a labelled second monoclonal antibody having the ability to bind an epitope on the IL-2 protein which is different than the epitope bound by said first monoclonal antibody;

(4) separating said solid carrier from said fluid sample and said unreacted labelled antibody; and

(5) measuring the amount of labelled antibody associated with said solid carrier; wherein the amount of said label present on said solid-phase substrate is proportional to the quantity of interleukin-2 protein present in said sample.

As would be recognized by skilled artisans, mABs from Group 1, 2 and 3 may be used interchangeably in the immunometric assay as long as the mABs don't bind to the same epitope on the IL-2 protein. For example, the Group 3 mAB, 13A6 could not be used with the Group 1, mAB, 13D2 as both mABs bind to the 41-55 epitope of the IL-2 protein. Preferred for use in the immunometric assay is the Group 1 mAB 5B1 plus the Group 2 mAB 17A1. Both of this mABs have affinities in the range of $1 \times 10^9$ M$^{-1}$.

The Two-Site Sandwich Assay of the invention has been standardized with a sensitivity of 0.5 to 1.0 ng/ml of IL-2 (32.25 to 64.5 fmoles/ml). The linear detecting range of the assay is 0.5-1.0 ng/ml to 10 ng/ml which corresponds to 5-10 U/ml to 100 U/ml. The sensitivity of the assay for IL-2 diluted in buffer containing bovine serum albumin and in 100% human serum is comparable.

Even greater sensitivity can be obtained if the Group 1 mAB, 5B1 is used in conjunction with the Group 2 mAB, 17A1 in the Assay. In this case, as little as 0.1mg of IL-2 per ml of sample (6.45f moles) can be detected. This corresponds to 2 Units of IL-2 activity per ml of sample.

As used herein the term "biological fluid" refers to blood, lymph, and the like which contains IL-2 protein. The preferred biological fluid for use in the immunometric assay of the invention is serum or plasma, and especially serum.

The first monoclonal antibody of the invention used in the immunometric assay described herein may be immobilized on any of the common supports used in immunometric assays. Among these may be mentioned, plastic beads or test tubes made from polyethylene, polystyrene, polypropylene or other suitable material. Also useful are particulate material such as agarose, cross-linked dextran, and other polysaccharides. The techniques for such bonding are well known to those skilled in the art. For example, antibodies may be bound to polysaccharide polymers using the process described in U.S. Patent No. 3,645,552.

The labelled second monoclonal antibody of the invention may be provided with any of the labels commonly used in prior art immunometric assays. Among these may be mentioned fluorogenic lables for detection by fluorimetry as described in U.S. Pat. No. 3,940,475 and enzymatic markers as described in U.S. Pat. No. 3,645,090 or radioisotopic labels I$^{125}$ using, for example, the procedure of Hunter and Greenwood, Nature (1962), 144:945. An enzymatic label is preferred for use herein.

## Example 8

### A. IL-2 Two-Site Sandwich Assay

The Group 1 mAB 5B1 was diluted to 20 μg/ml with Coating Buffer (50 mM Tris-HCl, 150 mMNaCl, pH 8.0) and 100μl of the solution was added to each well of a Costar EIA microtiter plate. The plates were incubated overnight at room temperature. Thereafter, the plates were washed three times with wash Buffer (25 mM Na phosphate buffer, 150 mMNaCl, 0.05% Tween 20). Prewarmed (37°C) Blocking Buffer (250μl) was added to each well of the prewashed plates. The plates were incubated 15-30 min. at 37°C and thereafter washed three times with Wash Buffer. Thereafter, 100μl of serum sample or standard was added to each well and the plate was incubated overnight at 4°C. The plate was washed 3$^\times$ with PBS/Tween buffer. To each well was then added 100μl of Biotinylated Antibody Diluent (10 or 20μg/ml of MAB 17A1) and the plate was incubated 2 hrs at 37°C with shaking. The plate was thereafter washed 3$^\times$ with PBS/Tween and 100μl of Streptavidin/peroxidase conjugate was added to each well. The plate was then incubated 15 min. at room temperature with shaking. The plate was washed 3$^\times$ with PBS/Tween and 100 μl

of Peroxidase Substrate was added to each well, incubated for 30 min. at room temperature with shaking. The reaction was stopped with 50μl of a solution containing 2.5M $H_2SO_4$ and 50mM sodium metabisulfite. The plate was read on a Titertak Multiscan MC at an optical density of 492.

## B. Preparation of Standards for Two-Site Assay

IL-2 was diluted in 1.4% bovine serum albumin (BSA) or in human serum. The solution was diluted to give a series of dilutions containing from 0.1μg/ml to 0.1 mg/ml. Eleven wells are used for the standard curve. One well is used for the buffer blank.

## C. Biotinylation of Monoclonal Antibody

The monoclonal antibody from Group 2 identified as 17A1 was dialyzed overnight against 0.1M sodium bicarbonate buffer pH 8.4 at 4°C (at least 2 × 2 liters). The protein concentration was adjusted to 1-5 mg/ml with 0.1M sodium bicarbonate buffer, pH 8.4. There was added 100μl of 1.1 mg/ml N-hydroxy succinimide ester of biotin in freshly prepared DMSO to each ml of antibody (1.0 mg). The reaction was allowed to proceed at room temperature for 2 hr. with occasionally shaking. Thereafter, any unreacted ester was blocked by adding 100μl of 1N $NH_4Cl$ and incubating the mixture for an additional 10 mins. Thereafter, 1 ml of 1% BSA in PBS buffer (1 ml for each mg of antibody biotinylated) is added and the reaction mixture was dialyzed overnight at 4°C against PBS (pH 7.4) containing 0.01% thimerosal (at least 2 liters of PBS should be used).

## D. Preparation of Peroxidase Substrate

A solution of o-phenylenediamine (0.4 mg/ml) was prepared in citrate buffer. To a 10 ml aliquot was added 4μl of 30% $H_2O_2$ to give a final concentration of 0.012%.

E. <u>Composition</u> of <u>Other</u> <u>Buffer</u> and <u>Diluents</u>

| | | |
|---|---|---|
| 1. | Coating buffer | 50 mM Tris-HCl pH 8.0<br>150mM NaCl |
| 2. | Blocking buffer - "FBS" buffer + 160 µg/ml human IgG" | 10% FBS (heat inactivated)<br>1% BSA<br>0.3% gelatin<br>25mM sodium phosphate<br>buffer pH 8.0<br>150mM NaCl<br>160µg/ml human IgG |
| 3. | IL-2 and Streptavidin-peroxidase diluent - "1.4% BSA Buffer" | 1.4% BSA<br>0.3% gelatin<br>25mM sodium phosphate<br>buffer pH 7.5<br>150mM NaCl |
| 4. | Biotinylated Antibody diluent - "FBS" Buffer + Tween + huIgG | "FBS" buffer + human IgG<br>0.2% Tween 20 |
| 5. | o-phenylenediamine buffer | 0.1M citrate buffer pH 4.5 |
| 6. | Wash buffer - "PBS/Tween" | 25mM sodium phosphate buffer<br>pH 7.2<br>150mM NaCl<br>0.05% Tween 20 |

The above-described Two-Site Sandwich Assay is highly specific and, thus, highly sensitive, because the potential for cross-reactivity has been eliminated or greatly reduced by the use of the monoclonal antibodies of the invention. Since the occurrence of cross-reactivity is reduced or eliminated, the prospect of "false - positive" results is greatly reduced or eliminated.

Monoclonal antibodies (mAB) specific for natural and recombinant IL-2 have been developed and tested for inhibition of IL-2 activity by an IL-2 Neutralization Assay and an IL-2 Receptor Binding Assay. The epitopes of 75 mABs have been identified by ELISA reactivity on IL-2 synthetic peptides and by Western blot and Dot blot analysis on recombinant IL-2 analogues. The mABs can be divided into 3 groups: Group 1 inhibit IL-2 activity in both functional assays, Group 2 do not inhibit IL-2 activity, and Group 3 inhibit mouse and human IL-2 activity but not rat IL-2 activity. Comparison of the epitope mapping data with inhibition of IL-2 activity highlights two epitopes which are important for human IL-2 activity: amino acids 1-19 and 41-55. Within the 41-55 area, antibody 13A6 which neutralizes mouse IL-2 activity has an epitope which must be different from, but close to, the epitope of 13D2 which does not bind or neutralize mouse IL-2. Evidence that the 1-19 and 40-60 sequences are physically adjacent in the native molecule comes from epitope competition studies. Antibody 5B1, which binds residues 1-12, inhibits a mAB specific for residues 40-70 from binding IL-2, mABs 17A1 and 3D5 which bind residues 21-123 and 71-87, respectively, are not blocked in the same assay. Of the 75 mABs analyzed, more than 95% recognize epitopes which are concentrated between amino acids 1-19 and 40-80.

14

**Claims**

1. A monoclonal antibody which has greater than $3.5 \times 10^7 M^{-1}$ affinity for native or recombinant human IL-2, which inhibits native or recombinant human IL-2 function to greater than 50% as measured by a conventional Neutralization Assay and Receptor Binding Assay and which binds said IL-2 protein between amino acids 1-12, 9-19, 21-123 or 41-55.

2. A monoclonal antibody according to Claim 1 which binds said IL-2 protein between amino acids 1-12 or 9-19.

3. A monoclonal antibody according to Claim 1 which binds said IL-2 between amino acids 41-55.

4. A monoclonal antibody according to Claim 1 which binds said IL-2 between amino acids 21-123.

5. A monoclonal antibody according to Claim 2 which has an affinity for human IL-2 of about $1.0 \times 10^9 M^{-1}$.

6. A monoclonal antibody which has greater than about $2.7 \times 10^7 M^{-1}$ affinity for native or recombinant human IL-2 protein as measured by conventional Radio Immunoassay, Assay, which does not inhibit IL-2 function as measured by conventional Neutralization Assay and Receptor Binding Assay and which binds said IL-2 protein between amino acids 40-70, 42-56, 52-70, 66-87, 71-87, 78-87, 107-121 or 21-123.

7. A monoclonal antibody according to Claim 6 which binds IL-2 protein between amino acids 40-70 or 42-56.

8. A monoclonal antibody according to Claim 6 which binds IL-2 protein between amino acids 66-87, 71-87 or 78-87.

9. A monoclonal antibody according to Claim 6 which binds IL-2 protein between amino acids 21-123 or 107-121.

10. A monoclonal antibody according to Claim 9 which has an affinity of about $1.0 \times 10^9 M^{-1}$.

11. A monoclonal antibody according to Claim 7 which has an affinity of about $2.7 \times 10^8 M^{-1}$.

12. A monoclonal antibody which inhibits native or recombinant human or mouse IL-2 as measured by Neutralization Assay and Receptor Binding Assay and which binds the epitope between amino acids 41-55 on the IL-2 protein, and which has an affinity for human or mouse IL-2 protein, when measured by Radio Immunoassay, of about $3.5 \times 10^7 M^{-1}$.

13. A monoclonal antibody according to Claim 12 wherein said antibody inhibits human IL-2 to greater than 40% and which inhibits mouse IL-2 to greater than 75% when measured by Neutralization Assay.

14. A method for the quantitative determination of IL-2 protein in a sample of biological fluid, which method comprises:

(1) Binding to a solid carrier, a first monoclonal antibody having the ability to bind to an epitope on said IL-2 protein;

(2) Contacting said fluid sample containing IL-2 protein with said first monoclonal antibody to form an insoluble complex of said first monoclonal antibody and said IL-2 protein;

(3) Contacting the complex of Step 2 with a known amount of a labelled second monoclonal antibody having the ability to bind to an epitope on said IL-2 protein;

(4) Separating said solid carrier from said fluid sample and unreacted labelled second monoclonal antibody; and

(5) Measuring the amount of said labelled second monoclonal antibody associated with said solid carrier;

wherein the amount of said label is proportional to the quantity of IL-2 protein present in said sample: wherein said first and second monoclonal antibodies each have an affinity for IL-2 protein of at least about $2.75 \times 10^7 M^{-1}$; and wherein said first monoclonal antibody and said second monoclonal antibody bind the IL-2 protein between amino acids, 1-12, 9-19, 21-123, 42-56, 40-70, 41-55, 78-87, 52-70, 66-87, 71-87 or 107-121; provided, that said first and said second monoclonal antibodies do not recognize the same or overlapping epitopes on the IL-2 protein.

15. A method according to claim 14 wherein said first and said second monoclonal antibody have an affinity for IL-2 protein of at least about $5.0 \times 10^7 M^{-1}$.

16. A method according to claim 14 wherein said first monoclonal antibody and said second monoclonal antibody have an affinity for IL-2 protein of at least about $2.8 \times 10^7 M^{-1}$.

17. A method according to claim 14 wherein said first and said second monoclonal antibody have an affinity for IL-2 protein of at least about $1.0 \times 10^9 M^{-1}$.

18. A method according to claim 17 wherein said first monoclonal antibody binds IL-2 protein between amino acids 1-12 and said second monoclonal antibody binds IL-2 protein between amino acids 21-123.

19. A method according to claim 14 wherein the labelled antibody is labelled with a member selected from the group consisting of a radioactive isotope, an enzyme, and a fluorescent material.

20. A method according to claim 19 wherein the labelled monoclonal antibody is labelled with an enzyme.